(19) Europäisches Patentamt
European Patent Office
Office européen des brevets

(11) Veröffentlichungsnummer: **0 373 545 B1**

(12) **EUROPÄISCHE PATENTSCHRIFT**

(45) Veröffentlichungstag der Patentschrift: **16.02.94**

(51) Int. Cl.$^5$: **C07K 99/56**, A61K 37/43

(21) Anmeldenummer: **89122757.1**

(22) Anmeldetag: **09.12.89**

Die Akte enthält technische Angaben, die nach dem Eingang der Anmeldung eingereicht wurden und die nicht in dieser Patentschrift enthalten sind.

# (54) Kompetitive Gonadoliberin-Antagonisten.

(30) Priorität: **14.12.88 DE 3842010**

(43) Veröffentlichungstag der Anmeldung:
**20.06.90 Patentblatt 90/25**

(45) Bekanntmachung des Hinweises auf die Patenterteilung:
**16.02.94 Patentblatt 94/07**

(84) Benannte Vertragsstaaten:
**AT BE CH DE ES FR GB GR IT LI LU NL SE**

(56) Entgegenhaltungen:
**EP-A- 0 097 031**
**EP-A- 0 263 521**
**WO-A-89/09786**
**NL-A- 7 413 081**

(73) Patentinhaber: **HOECHST AKTIENGESELLSCHAFT**

**D-65926 Frankfurt(DE)**

(72) Erfinder: **Kolar, Cenek, Dr.**
**Deutschhausstrasse 20**
**D-3550 Marburg(DE)**
Erfinder: **König, Wolfgang, Dr.**
**Eppsteiner Strasse 25**
**D-6238 Hofheim am Taunus(DE)**
Erfinder: **Sandow, Jürgen Kurt, Dr.**
**Am Haideplacken 22**
**D-6240 Königstein/Taunus(DE)**

**EP 0 373 545 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Natürlich vorkommende Gonadoliberine (Gn-RH) verschiedener Spezies sind Dekapeptide folgender Strukturen: h- , p-, o- Pgl-His-Trp-Ser-Tyr-Gly-Leu-Arg-Pro-Gly-$NH_2$
g-Gn-RH-I Pgl-His-Trp-Ser-Tyr-Gly-Leu-Gln-Pro-Gly-$NH_2$
g-Gn-RH-II Pgl-His-Trp-Ser-His-Gly-Trp-Tyr-Pro-Gly-$NH_2$
sa-Gn-RH Pgl-His-Trp-Ser-Tyr-Gly-Trp-Leu-Pro-Gly-$NH_2$
pe-Gn-RH Pgl-His-Tyr-Ser-Leu-Glu-Trp-Lys-Pro-Gly-$NH_2$
[h- (Mensch), p- (Schwein) o- (Schaf): Biochem. Biophys. Res. Commun. 43 (1971) 1334; g- (Huhn-I) South Africa J. Science 78 (1982) 124; g- (Huhn II): Proc. Natl. Acad. Sci. USA 81 (1984) 3874; sa- (Lachs): Proc. Natl. Acad. Sci. USA 80 (1983) 2794; pe- (Neunauge): J. Biol. Chem. 261 (1986) 4812-4819.]

Gn-RH wird bei Säugern hauptsächlich im Hypothalamus gebildet und bewirkt in der Hypophyse eine Ausschüttung von Lutropin (LH) und Follitropin (FSH).

Kompetitive Antagonisten des Gn-RH hemmen über die Blockierung der Gn-RH-Rezeptoren die Bildung von LH und FSH und damit auch die Synthese von Östrogen bei weiblichen Tieren bzw. Frauen oder Testosteron bei männlichen Tieren bzw. Männern. In der Literatur wurden bereits zahlreiche Gn-RH-Antagonisten beschrieben [J.J. Nestor, Jr. et al. In Publishers B.V. 1984, pp. 24-35; A.S. Dutta, Drugs of the Future 13 (1988) 761-787.], die meist eine basische Aminosäure in Position 6 enthalten. Durch diese basische Ladung in Position 6 werden die Peptide wasserlöslicher. Eine negative Begleiterscheinung dieser basischen Gruppe ist jedoch eine Histamin-ausschüttende Wirkung. Das "Nal-Glu", bei dem das Arg in position 5 versetzt wurde und in Position 6 D-4-p-Methoxybenzoyl-2-amino-buttersäure steht, hat eine stark reduzierte Histaminausschüttung [A. Phillips et al., Life Sci. 41 (1987) 2017-2022]. Geringer basische Substitutionen in Position 6, wie z.B. D-Nicotinoyl-lysin [K. Folkers et al., Z. Naturforsch. 42b (1987) 101-106; A. Ljungqvist et al., Biochem. Biophys. Res. Commun. 148 (1987) 849 856], D-Citrullin oder D-Homocitrullin [S. Bajusz et al. Proc. Natl. Acad. Sci. USA 85 (1988) 1637-1641] verringern ebenso die Histamin-Freisetzung.

In der EP-A 263521 (HOE 86/F 253) wurde durch Substitution mit glycosylierten Zuckern sowohl Gn-RH-Agonisten als auch Gn-RH-Antagonisten mit günstigen Eigenschaften erhalten. Zum einen konnte dadurch die Wasserlöslichkeit erhöht werden und zum andern die anaphylaktische Wirkung, die vor allem bei Gn-RH-Antoginsten zu beobachten war, gesenkt werden.

In weiterer Bearbeitung diese glycosylierten Gn-RH-Derivate fanden wir überraschenderweise, daß speziell Verbindungen der allgemeinen Formel I besonders stark endogenes Gn-RH antagonisieren und deshalb den Serum-Spiegel des luteotropen Hormons (LH), des follikelstimulierenden Hormons (FSH) des Testosterons und des Östrogens senken.

Gegenstand der Erfindung sind Peptide der allgemeinen Formel I

Ac-D-Nal(2)-D-Phe-D-Phe-Ser-X-D-Ser(Rha)-Leu-Arg-Pro-Y (I),

in welcher
Ac für Acetyl,
D-Nal(2) für 3-(2-Naphtyl)-D-alanin,
D-Phe für D-Phenylalanin,
Ser für L-Serin,
X für L-Tyrosin (Tyr) oder L-Histidin (His),
D-Ser(Rha) für O- ($\alpha$-L-Rhamnopyranosyl)-D-serin,
Leu für L-Leucin,
Arg für L-Arginin,
Pro für L-Prolin und
Y für Glycin-amid (Gly-$NH_2$), D-Alanin-amid (D-Ala-$NH_2$), Azaglycin-amid (Azgly-$NH_2$) oder NH-$C_2H_5$ steht,
sowie deren physiologisch verträgliche Salze.

Besonders bevorzugt als Antagonisten sind Verbindungen, bei denen für X Tyr oder His und für Y Azgly-$NH_2$ oder D-Ala-$NH_2$ steht.

D-Phe in Position 2 und 3 anstatt D-pCl-$Phe^2$ und D-$Trp^3$ bei Detirelix [J.J. Nestor Jr., J. Med. Chem. 31 (1988) 65-72] oder D-pCl $Phe^2$ und D-3-Pyridyl-alanin in Position 3 bei "Nal-Glu" hat den Vorteil billiger zu sein. Wegen der unproblematischeren Chemie des D-Phe gestaltet sich auch die Synthese einfacher (weniger Nebenprodukte) und die Präparate sind stabiler.

Die Kombination D-Phe[2], D-Phe[3] wurde bereits in dem Antagonisten Ac-D-Nal(2)-D-Phe-D-Phe-Ser-Tyr-D-Arg-Phe-Arg-Pro-D-Ala-$NH_2$ eingesetzt, wo sie gegenüber der Verbindung mit D-pCl-Phe[2] und D-Trp[3] eine etwas geringere antiovulatorische Wirkung zeigte [S.J. Hocart et al., J. Med. Chem. 30 (1987) 735-739].

Die Peptide können unter Benutzung der allgemeinen Methoden der Peptidchemie (Houben-Weyl, Methoden der Organischen Chemie, Band 15) stufenweise vom C-terminalen Ende oder durch Segment-Kondensation hergestellt werden, z. B. durch Fragmentkondensation, dadurch gekennzeichnet, daß man ein Fragment mit N-terminaler freier Aminogruppe kondensiert, mit einem Fragment mit C-terminaler freier Carboxylgruppe, eine oder mehrere zum Schutz funktioneller Gruppen gegebenenfalls temporär eingeführte Schutzgruppen abspaltet und das so erhaltene Peptid gegebenenfalls in sein physiologisch verträgliches Salz überführt. Eine mögliche Synthese der Serin-glycoside ist in EP-A 263521 beschriebenen.

Um bei der Segmentkondensation die mögliche Racemisierung möglichst niedrig zu halten wird hier bevorzugt mit Dicyclohexylcarbodiimid (DCC) unter Zusatz von 3-Hydroxy-4-oxo-3,4-dihydro-1,2,3-benzotriazin (HOObt) gearbeitet. Als Amino-Schutzgruppen werden bevorzugt der durch katalytische Hydrierung abspaltbare Z-Rest oder der durch sekundäre Amine abspaltbare Fmoc-Rest herangezogen. Der Imidazol-Ring des Histidins wird vorzugsweise durch den 2,4-Dinitrophenyl-(Dnp)-Rest geschützt, der durch Mercaptane oder Hydrazin abgespalten werden kann.

Als besonders günstig erwies sich eine Segment-Kupplung nach dem Schema

(1-4) + (5-10) → (1-10).

Die Synthese wird durch nachfolgende 2 Reaktions-Schemata illustriert.

SCHEMA 1

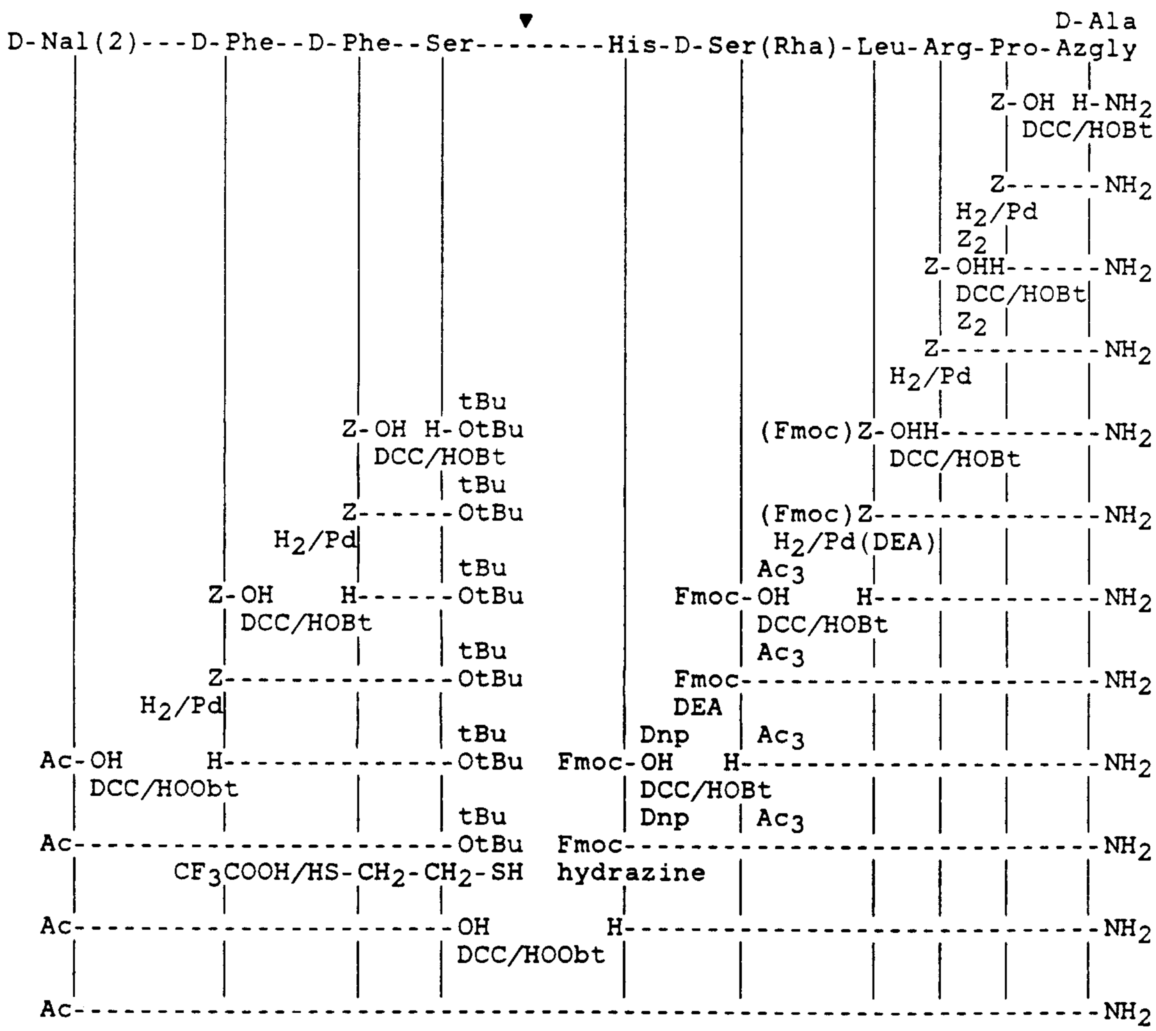

SCHEMA 2

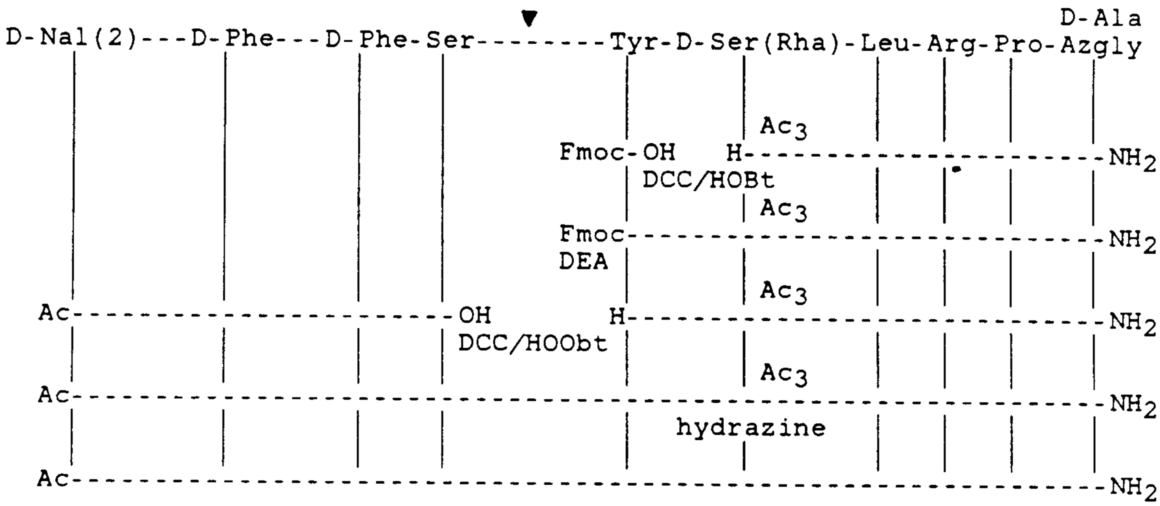


Die erfindungsgemäßen Gn-RH-Antagonisten können wie hochdosierte Gn-RH-Agonisten bei Gonadotropin- und Steroidabhängigen Erkrankungen eingesetzt werden. Der Vorteil der Antagonisten gegenüber den Agonisten ist jedoch, daß die primäre Stimulierungsphase der Agonisten vermieden wird.

Die bevorzugten Anwendungsformen beim Menschen ist die intranasale Applikation oder die Verwendung von Implantaten, da die Resorption aus dem Magen-Darm-Trakt nur gering ist und eine tägliche parenterale Anwendung für den Patienten unzweckmäßig ist.

Mittels eines Dosierzerstäubers werden über eine Spraydüse etwa 0,02-0,2 ml einer Pufferlösung, in der die erforderliche Menge des Wirkstoffs gelöst ist, in die Nase gesprüht. Bei einer parenteralen Applikation können die Dosierungen gegenüber der Intranasal Dosis um etwa eine Zehnerpotenz gesenkt werden.

Die erfindungsgemäßen Antagonisten werden beim erwachsenen Menschen intranasal in Dosen von 1-10 mg appliziert. Die Einzeldosis in Implantaten beträgt etwa 5-50 mg für einen Zeitraum von jeweils 4-8 Wochen. Parenteral appliziert, genügen bereits 0,1-1 mg Applikation.

Die erfindungsgemäßen Peptide wurden an männlichen Ratten mittels Dauerinfusion (MINIPUMPS) auf athrophische Wirkung an androgenanhängigen Organen und auf LH- und Testosteronsenkende Wirkung im Serum und Blut geprüft.

Am wirksamsten waren die Verbindungen der Beispiele 1 und 2. Die Verbindung von Beispiel 3 ist noch stark wirksam, während die Verbindung von Beispiel 4 aus dieser Gruppe die schwächste Wirkung zeigte.

Weitere verwendete Abkürzungen:

| | |
|---|---|
| HOBt | 1-Hydroxybenzotriazol |
| DEA | Diethylamin |

**Beispiel 1**

Ac-D-Nal(2)-D-Phe-D-Phe-Ser-His-D-Ser(Rha)-Leu-Arg-Pro-Azgly-$NH_2$

1a) Z-D-Phe-Ser(tBu)-OtBu

Zu einer Lösung von 21,2 g Z-D-Phe-OH, 9,6 g HOBt und 17,9 g HCl•H-Ser(tBu) OtBu in 150 ml Dimethylformamid gibt man bei 0 °C 9,07 ml N-Ethylmorpholin und 16,05 g DCC. Man läßt eine Stunde bei 0 °C rühren und über Nacht bei Raumtemperatur stehen. Der Niederschlag wird abgesaugt und das Filtrat eingeengt. Der Rückstand wird zwischen Essigester und Wasser verteilt. Die Essigesterphase wird nacheinander mit gesättigter $NaHCO_3$-Lösung, $KHSO_4/K_2SO_4$-Lösung und Wasser ausgeschüttelt, über $Na_2SO_4$ getrocknet und eingeengt. Nach Verreiben mit Petrolether kristallisiert die Substanz.
Ausbeute: 23,4 g
Schmp. 79-81 °C,
$[\alpha]_D^{21}$ = +20,6° (c=1, in Methanol)

1b) H-D-Phe-Ser(tBu)-OtBu•HCl

22,0 g Z-D-Phe-Ser(tBu)-OtBu werden in Methanol gelöst und unter Zugabe von methanolischer Salzsäure mittels eines Autotitrators bei pH 4,5 katalytisch (Pd/Kohle) hydriert. Nach Beendigung der Hydrierung wird der Katalysator über Kieselgur abgesaugt und das Filtrat eingeengt. Der Rückstand wird mit Diethylether verrieben. Die Substanz wird fest und kann abgesaugt werden.
Ausbeute: 15,6 g
Schmp. 154-156 °C
$[\alpha]_D^{22}$ = -24,7° (c=1, in Methanol)

1c) Z-D-Phe-D-Phe-Ser(tBu)-OtBu

Zu einer Lösung von 10,69 g Z-D-Phe-OH, 14,5 g H-D-Phe-Ser(tBut)-OtBu•HCl und 4,89 g HOBt in 150 ml Dimethylformamid gibt man bei 0 °C 4,71 ml N-Ethylmorpholin und 7,98 g DCC. Man läßt 1 Stunde bei 0 °C rühren und über Nacht bei Raumtemperatur stehen. Der Niederschlag wird abgesaugt und das Filtrat eingeengt. Der Rückstand wird zwischen Essigester und Wasser verteilt. Die Essigesterphase wird nacheinander mit gesättigter $NaHCO_3$-Lösung, $KHSO_4/K_2SO_4$-Lösung und Wasser ausgeschüttelt, über $Na_2SO_4$ getrocknet und eingeengt. Das Öl kristallisiert aus Isopropanol/Petrolether.
Ausbeute 19,2 g
Schmp. 91-92 °C
$[\alpha]_D^{22}$ = +27,5° (c=1, in Methanol)

1d) H-D-Phe-D-Phe-Ser(tBu)-OtBu•HCl

18,0 g Z-D-Phe-D-Phe-Ser(tBu) werden wie in Beispiel 1b) in Methanol gelöst und katalytisch hydriert. Der Rückstand wird mit Diethylether verrieben und abgesaugt.
Ausbeute 13,15 g
Schmp. 143-144 °C
$[\alpha]_D^{23}$ -2,7° (c=1, in Methanol)

1e) Ac-D-Nal(2)-D-Phe-D-Phe-Ser(tBu)-OtBu

Zu einer Lösung von 11 g HCl•H-D-Phe-D-Phe-Ser(tBu)-OtBu,5,16 g Ac-D-Nal(2)-OH und 3,28 g HOObt in 150 ml Dimethylformamid gibt man bei 0 °C 2,56 ml N-Ethyl-morpholin und 4,4 g DCC und läßt 1 Stunde bei 0 °C rühren und über Nacht bei Raumtemperatur stehen. Der Niederschlag wird abgesaugt und das Filtrat eingeengt. Der Rückstand wird wie bei Beispiel 1a) aufgearbeitet.
Ausbeute: 10,56 g
Schmp. 187 °C
$[\alpha]_D^{20}$ = +11,0° (c=1, in Methanol)

1f) Ac-D-Nal(2)-D-Phe-D-Phe-Ser-OH

10 g Ac-D-Nal(2)-D-Phe-D-Phe-Ser(tBu)-OtBu werden in einer Mischung aus 40 ml 90 %iger wäßriger Trifluoressigsäure und 1,6 ml 1,2-Dimercaptoethan gelöst. Man läßt eine Stunde bei Raumtemperatur stehen und engt ein. Der Rückstand wird mit Wasser verrieben und im Hochvakuum getrocknet.
Ausbeute: 9,84 g
$[\alpha]_D^{23}$ = +14,2° (c=1, in Methanol)

1g) Ac-D-Nal(2)-D-Phe-D-Phe-Ser-His-D-Ser(Rha)-Leu-Arg-Pro-Azgly-$NH_2$

Zu einer Lösung von 320 mg Ac-D-Nal(2)-D-Phe-D-Phe-Ser-OH, 424,2 mg H-His-D-Ser(Rha)-Leu-Arg-(HCl)-Pro-Azgly-$NH_2$ und 81,5 mg HOObt in 7 ml Dimethylformamid gibt man bei 0 °C 110 mg DCC. Man rührt 1 Stunde bei 0 °C und läßt über Nacht bei Raumtemperatur stehen. Der Niederschlag wird abgesaugt und das Filtrat eingeengt. Der Rückstand wird in 100 ml Pentanol aufgenommen und dreimal mit gesättigter $NaHCO_3$-Lösung extrahiert. Die Pentanolphase wird mit 1N HCl neutralisiert und eingeengt. Der Rückstand wird mit Essigester verrieben und abgesaugt.
Ausbeute: 580 mg

Die Substanz wird in 120 ml 10 %iger Essigsäure gelöst. Man filtriert die Lösung über 40 ml eines schwach basischen Ionenaustauschers (Acetat-Form) und eluiert mit Wasser. Die Eluat-Fraktionen, die das Peptid enthalten, werden vereinigt und gefriergetrocknet.
Ausbeute: 468 mg
Die oben gewonnenen 468 mg Rohsubstanz werden an einem alkylierten Dextrangel chromatographisch gereinigt. Als Elutionsmittel diente eine Mischung aus 4300 ml Wasser, 430 ml n-Butanol und 350 ml Eisessig.
Ausbeute: 276 mg
$[\alpha]_D^{22}$ = -53,2° (c = 1, in Wasser)
Gehalt an Peptidbase: 77,7 %

**Beispiel 2**

Ac-D-Nal-D-Phe-D-Phe-Ser-His-D-Ser(Rha)-Leu-Arg-Pro-D-Ala-$NH_2$-acetat

Zu einer Lösung von 320 mg AC-D-Nal-D-Phe-D-Phe-Ser-OH, 499 mg H-His-D-Ser(Rha)-Leu-Arg-Pro-D-Ala-$NH_2$-tosylat und 81,5 mg HOObt in 7 ml Dimethylformamid gibt man bei 0 °C 110 mg DCC. Man läßt 1 Stunde bei 0 °C und anschließend bei Raumtemperatur rühren. Anderntags wird der Niederschlag abgesaugt und das Filtrat eingeengt. Der Rückstand wird zwischen Pentanol und gesättigter $NaHCO_3$-Lösung verteilt. Die Pentanolphase wird mit $NaHCO_3$-Lösung und Wasser gewaschen und im Hochvakuum eingeengt. Der Rückstand wird mit Essigester verrieben, abgesaugt und getrocknet.
Ausbeute: 650 mg
Oben gewonnene Substanz wird in ca. 40 ml 10 %iger Essigsäure gelöst, von Unlöslichem filtriert und über 40 ml eines schwach basischen Ionenaustauschers (in Acetatform) chromatographiert. Es wird mit Wasser eluiert. Die Fraktionen, die Substanz enthielten wurden vereinigt und gefriergetrocknet.
Ausbeute: 460 mg
Reinigung analog Beispiel 1g).
Ausbeute: 285 mg
$[\alpha]_D^{23}$ = -52,6° (c = 1, in Wasser)
Gehalt an Peptidbase: 92 %

**Beispiel 3**

Ac-D-Nal-D-Phe-D-Phe-Ser-Tyr-D-Ser(Rha)-Leu-Arg-Pro-Azgly-$NH_2$

3a) H-Tyr-D-Ser(Rha)-Leu-Arg-Pro-Azgly-$NH_2$ • HCl

Zu einer Lösung von 1 g H-Tyr-D-Ser[Rha($Ac_3$)]-Leu-Arg-Pro-Azgly-$NH_2$ • HCl in 10 ml Dimethylacetamid gibt man 1 ml Hydrazinhydrat und rührt 4 Stunden bei Raumtemperatur. Anschließend engt man die klare Lösung ein und verreibt den Rückstand mit Diethylether und Methyl-tert.-butylether, saugt ab und trocknet.
Ausbeute: 0,9 g
$[\alpha]_D^{22}$ = -38,8° (c = 1, in Methanol)

3b) Ac-D-Nal-D-Phe-D-Phe-Ser-Tyr-D-Ser(Rha)-Leu-Arg-Pro-Azgly-$NH_2$-acetat

Zu einer Lösung von 320 mg Ac-D-Nal-D-Phe-D-Phe-Ser-OH, 419 mg H-Tyr-D-Ser(Rha)-Leu-Arg-Pro-Azgly-$NH_2$ • HCl und 81,5 mg HOObt in 7 ml Dimethylformamid gibt man bei 0 °C 110 mg DCC. Man rührt 1 Stunde bei 0 °C und über Nacht bei Raumtemperatur. Der Niederschlag wird abgesaugt und das Filtrat eingeengt. Der Rückstand wird zwischen 400 ml Pentanol und 100 ml gesättigter $NaHCO_3$-Lösung verteilt. Von Unlöslichem (2. Niederschlag: enthält bereits gewünschte Substanz) wird abgesaugt. Die Pentanolphase wird mit gesättigter $NaHCO_3$-Lösung und Wasser gewaschen und eingeengt. Der Rückstand wird mit dem 2. Niederschlag vereinigt (Ausbeute: 375 mg) und auf Kieselgel chromatographisch gereinigt. Als Elutionsmittel diente ein Gemisch von Methylenchlorid : Methanol : Wasser : Essigsäure wie 70 : 40 : 3 : 3.
Ausbeute: 118 mg
$[\alpha]_D^{21}$ -96,9° (c = 1, in Wasser)
Gehalt an Peptidbase: 70 %

**Beispiel 4**

Ac-D-Nal-D-Phe-D-Phe-Ser-Tyr-D-Ser(Rha)-Leu-Arg-Pro-NH-$C_2H_5$

4a) Fmoc-Tyr-D-Ser[Rha($Ac_3$)]-Leu-Arg-Pro-NH-$C_2H_5$

Zu einer Lösung von 3,23 g Fmoc-Tyr-OH, 7,54 g H-D-Ser[Rha($Ac_3$)]-Leu-Arg-Pro-NH-$C_2H_5$-tosylat und 1,1 g HOBt in 40 ml Dimethylformamid gibt man bei 0 °C 1,76 g DCC. Man läßt 1 Stunde bei 0 °C und über Nacht bei Raumtemperatur rühren. Der Niederschlag wird abgesaugt und das Filtrat eingeengt. Der Rückstand wird zwischen n-Pentanol und gesättigter $NaHCO_3$-Lösung verteilt. Die Pentanolphase wird mit gesättigter $NaHCO_3$-Lösung und Wasser ausgeschüttelt, mit 1N methanolischer p-Toluolsulfonsäure neutralisiert und eingeengt. Der Rückstand wird mit Methyl-tert.-butylether verrieben und abgesaugt.
Ausbeute: 10,5 g
$[\alpha]_D^{21}$ = -40,6° (c=1, in Methanol)

4b) H-Tyr-D-Ser[Rha($Ac_3$)]-Leu-Arg-Pro-NH-$C_2H_5$-tosylat

Zu einer Lösung von 9,96 g Fmoc-Tyr-D-Ser[Rha($Ac_3$)]-Leu-Arg-Pro-NH-$C_2H_5$ in 30 ml Dimethylformamid gibt man bei Raumtemperatur 7,5 ml Diethylamin. Man rührt 15 Minuten bei Raumtemperatur und engt ein. Der Rückstand wird mit Diethylether verrieben und abgesaugt.
Ausbeute: 8,6 g
$[\alpha]_D^{23}$ = -51,6° (c=1, in Methanol)

4c) Ac-D-Nal-D-Phe-D-Phe-Ser-Tyr-D-Ser(Rha)-Leu-Arg-Pro-NH-$C_2H_5$-acetat

Zu einer Lösung von 320 mg Ac-D-Nal-D-Phe-D-Phe-Ser-OH, 404 mg H-Tyr-D-Ser(Rha)-Leu-Arg-Pro-NH-$C_2H_5$-tosylat und 81,5 mg HOObt in 7 ml Dimethylformamid gibt man bei 0 °C 110 mg DCC. Man läßt 1 Stunde bei 0 °C und anschließend über Nacht bei Raumtemperatur rühren. Der Niederschlag wird abgesaugt und das Filtrat eingeengt. Der Rückstand wird analog Beispiel 2 aufgearbeitet (Ausbeute 420 mg) und analog Beispiel 3 gereinigt.
Ausbeute: 153 mg
$[\alpha]_D^{23}$ = -100,8° (c=1, in Wasser)
Gehalt an Peptidbase: 76 %

**Patentansprüche**
**Patentansprüche für folgende Vertragsstaaten : AT, BE, CH, DE, FR, GB, IT, LI, LU, NL, SE**

**1.** Peptid der allgemeinen Formel I
Ac-D-Nal(2)-D-Phe-D-Phe-Ser-X-D-Ser(Rha) Leu-Arg-Pro-Y (I),
in welcher
Ac für Acetyl,
D-Nal(2) für 3-(2-Naphtyl)-D-alanin,
D-Phe für D-Phenylalanin,
Ser für L-Serin,
X für L-Tyrosin (Tyr) oder L-Histidin (His),
D-Ser(Rha) für O-(α-L-Rhamnopyranosyl)-D-serin,
Leu für L-Leucin,
Arg für L-Arginin,
Pro für L-Prolin und
Y für Glycin-amid (Gly-$NH_2$), D-Alanin-amid (D-Ala-$NH_2$), Azaglycin-amid (Azgly-$NH_2$) oder NH-$C_2H_5$ steht,
sowie dessen physiologisch verträgliche Salze.

**2.** Peptid der allgemeinen Formel I gemäß Anspruch 1, in welcher
X Tyr oder His und
Y Azgly-$NH_2$ oder D-Ala-$NH_2$ bedeuten,
sowie dessen physiologisch verträgliche Salze.

3. Verfahren zur Herstellung eines Peptids der allgemeinen Formel I gemäß einem oder mehreren der Ansprüche 1 bis 2, dadurch gekennzeichnet, daß man ein Fragment mit N-terminaler freier Aminogruppe kondensiert mit einem Fragment mit C-terminaler freier Carboxylgruppe, eine oder mehrere zum Schutz funktioneller Gruppen gegebenenfalls temporär eingeführte Schutzgruppen abspaltet und das so erhaltene Peptid gegebenenfalls in sein physiologisch vertägliches Salz überführt.

4. Peptid der allgemeinen Formel I gemäß einem oder mehreren der Ansprüche 1 bis 2 zur Anwendung als Heilmittel.

5. Verwendung eines Peptids der allgemeinen Formel I gemäß einem oder mehreren der Ansprüche 1 bis 2 zur Behandlung Gonadoliberin-, Gonadotropin- und Steroid-abhängiger Erkrankungen.

6. Pharmazeutische Zübereitung enthaltend ein Peptid der allgemeinen Formel I gemäß einem oder mehreren der Ansprüche 1 bis 2 oder dessen physiologische verträgliches Salz.

**Patentansprüche für folgende Vertragsstaaten : ES, GR**

1. Verfahren zur Herstellung eines Peptids der allgemeinen Formel I
Ac-D-Nal(2)-D-Phe-D-Phe-Ser-X-D-Ser(Rha)-Leu-Arg-Pro-Y (I),
in welcher
Ac für Acetyl,
D-Nal(2) für 3-(2-Naphtyl)-D-alanin,
D-Phe für D Phenylalanin,
Ser für L-Serin,
X für L-Tyrosin (Tyr) oder L-Histidin (His),
D-Ser(Rha) für O- ($\alpha$-L-Rhamnopyranosyl)-D-serin,
Leu für L-Leucin,
Arg für L-Arginin,
Pro für L-Prolin und
Y für Glycin-amid (Gly-$NH_2$), D-Alanin-amid (D-Ala-$NH_2$), Azaglycin amid (Azgly-$NH_2$) oder NH-$C_2H_5$ steht,
sowie dessen physiologisch verträgliche Salze,
dadurch gekennzeichnet, daß man ein Fragment mit N-terminaler freier Aminogruppe kondensiert mit einem Fragment mit C-terminaler freier Carboxylgruppe, eine oder mehrere zum Schutz funktioneller Gruppen gegebenenfalls temporär eingeführte Schutzgruppen abspaltet und das so erhaltene Peptid gegebenenfalls in sein physiologisch verträgliches Salz überführt.

2. Verfahren gemäß Anspruch 1, dadurch gekennzeichnet, daß ein Peptid der allgemeinen Formel I hergestellt wird in welcher
X Tyr oder His und
Y Azgly-$NH_2$ oder D-Ala-$NH_2$ bedeuten.

3. Verwendung eines nach einem der Ansprüche 1 und/oder 2 hergestellten Peptids der allgemeinen Formel I zur Behandlung Gonadoliberin-, Gonadotropin- und Steroid-abhängiger Erkrankungen.

4. Verfahren zur Herstellung einer pharmazeutischen Zübereitung enthaltend ein nach einem der Verfahren der Ansprüche 1 und/oder 2 hergestelltes Peptid der allgemeinen Formel I, dadurch gekennzeichnet, daß man dieses zusammen mit einem physiologisch unbedenklichen Träger und gegebenenfalls weiteren Hilfs- und Zusatzstoffen in eine geeignete Darreichungsform bringt.

**Claims**
**Claims for the following Contracting States : AT, BE, CH, DE, FR, GB, IT, LI, LU, NL, SE**

1. A peptide of the formula I Ac-D-Nal(2)-D-Phe-D-Phe-Ser-X-D-Ser(Rha)-Leu-Arg-Pro-Y (I),
in which
Ac represents acetyl,
D-Nal(2) represents 3-(2-naphthyl)-D-alanine,
D-Phe represents D-phenylalanine,

Ser represents L-serine,
X represents L-tyrosine (Tyr) or L-histidine (His),
D-Ser(Rha) represents O-(α-L-rhamnopyranosyl)-D-serine,
Leu represents L-leucine,
Arg represents L-arginine,
Pro represents L-proline and
Y represents glycinamide (Gly-$NH_2$), D-alaninamide (D-Ala-$NH_2$), azaglycinamide (Azgly-$NH_2$) or NH-$C_2H_5$,
as well as the physiologically tolerated salts thereof.

**2.** A peptide of the formula I as claimed in claim 1, in which X denotes Tyr or His and
Y denotes Azgly-$NH_2$ or D-Ala-$NH_2$,
as well as the physiologically tolerated salts thereof.

**3.** A process for the preparation of a peptide of the formula I as claimed in one or more of claims 1 to 2, which comprises condensing a fragment with an N-terminal free amino group with a fragment with a C-terminal free carboxyl group, eliminating one or more protective groups temporarily introduced where appropriate to protect functional groups, and converting the peptide obtained in this way into its physiologically tolerated salt where appropriate.

**4.** A peptide of the formula I as claimed in one or more of claims 1 to 2 for use as a medicine.

**5.** The use of a peptide of the formula I as claimed in one or more of claims 1 to 2 for the treatment of gonadoliberin-, gonadotropin- and steroid-dependent diseases.

**6.** A pharmaceutical composition containing a peptide of the formula I as claimed in one or more of claims 1 to 2 or the physiologically tolerated salt thereof.

**Claims for the following Contracting States : ES, GR**

**1.** A process for the preparation of a peptide of the formula I
Ac-D-Nal(2)-D-Phe-D-Phe-Ser-X-D-Ser(Rha)-Leu-Arg-Pro-Y (I),
in which
Ac represents acetyl,
D-Nal(2) represents 3-(2-naphthyl)-D-alanine,
D-Phe represents D-phenylalanine,
Ser represents L-serine,
X represents L-tyrosine (Tyr) or L-histidine (His),
D-Ser(Rha) represents O-(α-L-rhamnopyranosyl)-D-serine,
Leu represents L-leucine,
Arg represents L-arginine,
Pro represents L-proline and
Y represents glycinamide (Gly-$NH_2$), D-alaninamide (D-Ala-$NH_2$), azaglycinamide (Azgly-$NH_2$) or NH-$C_2H_5$,
as well as the physiologically tolerated salts thereof, which comprises condensing a fragment with an N-terminal free amino group with a fragment with a C-terminal free carboxyl group, eliminating one or more protective groups temporarily introduced where appropriate to protect functional groups, and converting the peptide obtained in this way into its physiologically tolerated salt where appropriate.

**2.** The process as claimed in claim 1, which comprises preparing a peptide of the formula I in which X denotes Tyr or His and Y denotes Azgly-$NH_2$ or D-Ala-$NH_2$.

**3.** The use of a peptide of the formula I prepared as claimed in either of claims 1 and 2 for the treatment of gonadoliberin-, gonadotropin- and steroid-dependent diseases.

**4.** A process for the preparation of a pharmaceutical composition containing a peptide of the formula I prepared by the process as claimed in either of claims 1 and 2, which comprises bringing this peptide, together with a physiologically acceptable vehicle and where appropriate further auxiliaries and

additives, into a suitable administration form.

**Revendications**

**Revendications pour les Etats contractants suivants : AT, BE, CH, DE, FR, GB, IT, LI, LU, NL, SE**

1. Peptide de formule générale I
   Ac-D-Nal(2)-D-Phe-D-Phe-Ser-X-D-Ser(Rha)-Leu-Arg-Pro-Y (I)
   dans laquelle

| | |
|---|---|
| Ac | représente le groupe acétyle, |
| D-Nal(2) | représente la 3-(2-naphtyl)-D-alanine, |
| D-Phe | représente la D-phénylalanine, |
| Ser | représente la L-sérine, |
| X | représente la L-tyrosine (Tyr) ou la L-histidine (His), |
| D-Ser(Rha) | représente l'O-($\alpha$-L-rhamnopyrannosyl)-D-sérine, |
| Leu | représente la L-leucine, |
| Arg | représente la L-arginine, |
| Pro | représente la L-proline et |
| Y | représente le glycine-amide (Gly-$NH_2$), le D-alanine-amide (D-Ala-$NH_2$), l'azaglycine-amide (Azgly-$NH_2$) ou NH-$C_2H_5$, |

   et sels physiologiquement acceptables de celui-ci.

2. Peptide de formule générale I selon la revendication 1, dans lequel
   X représente Tyr ou His et
   Y représente Azgly-$NH_2$ ou D-Ala-$NH_2$,
   et sels physiologiquement acceptables de celui-ci.

3. Procédé pour la préparation d'un peptide de formule générale I selon une ou plusieurs des revendications 1 et 2, caractérisé en ce que l'on condense un fragment à groupe amino N-terminal libre avec un fragment à groupe carboxy C-terminal libre, on élimine un ou plusieurs groupes protecteurs éventuellement introduits temporairement pour la protection de groupes fonctionnels, et éventuellement on convertit le peptide ainsi obtenu en un de ses sels physiologiquement acceptables.

4. Peptide de formule générale I selon une ou plusieurs des revendications 1 et 2, pour utilisation en tant que médicament.

5. Utilisation d'un peptide de formule générale I selon une ou plusieurs des revendications 1 et 2, dans le traitement de maladies dépendantes de la gonadolibérine, de la gonadotropine et de stéroïdes.

6. Composition pharmaceutique contenant un peptide de formule générale I selon une ou plusieurs des revendications 1 et 2 ou un sel physiologiquement acceptable de celui-ci.

**Revendications pour les Etats contractants suivants : ES, GR**

1. Procédé pour la préparation d'un peptide de formule générale I
   Ac-D-Nal(2)-D-Phe-D-Phe-Ser-X-D-Ser(Rha)-Leu-Arg-Pro-Y (I)
   dans laquelle

| | |
|---|---|
| Ac | représente le groupe acétyle, |
| D-Nal(2) | représente la 3-(2-naphtyl)-D-alanine, |
| D-Phe | représente la D-phénylalanine, |
| Ser | représente la L-sérine, |
| X | représente la L-tyrosine (Tyr) ou la L-histidine (His), |
| D-Ser(Rha) | représente l'O-($\alpha$-L-rhamnopyrannosyl)-D-sérine, |
| Leu | représente la L-leucine, |
| Arg | représente la L-arginine, |
| Pro | représente la L-proline et |
| Y | représente le glycine-amide (Gly-$NH_2$), le D-alanine-amide (D-Ala-$NH_2$), l'azaglycine-amide (Azgly-$NH_2$) ou NH-$C_2H_5$, |

   ainsi que de ses sels physiologiquement acceptables, caractérisé en ce que l'on condense un fragment

à groupe amino N-terminal libre avec un fragment à groupe carboxy C-terminal libre, on élimine un ou plusieurs groupes protecteurs éventuellement introduits temporairement pour la protection de groupes fonctionnels, et éventuellement on convertit le peptide ainsi obtenu en un de ses sels physiologiquement acceptables.

**2.** Procédé selon la revendication 1, caractérisé en ce que l'on prépare un peptide de formule générale I dans lequel
X représente tyr ou His, et Y représente Azgly-$NH_2$ ou D-Ala-$NH_2$.

**3.** Utilisation d'un peptide de formule générale I, préparé selon une ou plusieurs des revendications 1 et/ou 2, dans le traitement de maladies dépendantes de la gonadolibérine, de la gonadotropine et de stéroïdes.

**4.** Procédé pour la préparation d'une composition pharmaceutique contenant un peptide de formule générale I, préparé selon l'un des procédés des revendications 1 et/ou 2, caractérisé en ce qu'on le met sous une forme d'administration appropriée, conjointement avec un véhicule physiologiquement acceptable et éventuellement d'autres additifs et adjuvants.